# EUROPEAN PATENT APPLICATION

(11) **EP 1 475 088 A1**
(43) Date of publication of application: **10.11.2004**
(21) Application number: 03010123.2
(22) Date of filing: 05.05.2003
(51) Int. Cl.: A61K 31/192, A61K 31/426, A61P 25/14, A61P 25/30, A61P 27/12, A61P 29/00

(54) **Use of kynurenine-3-hydroxylase inhibitors for the preparation of medicaments for the treatment of l-dopa induced movement disorders, dyskinesias, drug addiction, pain and cataract**

(71) Applicant: Newron Pharmaceuticals S.p.A., 20091 Bresso (MI) (IT)
(72) Inventor: Salvati, Patricia, 20020 Arese (MI) (IT); Izzo, Emanuela, 20091 Bresso (MI) (IT)
(74) Representative: Minoja, Fabrizio, Dr.

(57) **Abstract**

Use of kynurenine-3-hydroxylase inhibitors for the preparation of medicaments for the treatment of L-DOPA induced movement disorders, dyskinesias, drug addiction, pain and cataract.

## Description

The present invention relates to the use of kynurenine-3-hydroxylase inhibitors for the preparation of medicaments for the treatment of L-DOPA induced movement disorders, dyskinesias, drug addiction, pain and cataract.

### BACKGROUND OF THE INVENTION

The kynurenine pathway is a major route of L-tryptophan metabolism. It generates compounds with neurotoxic oxidative activity and compounds that can modulate activity at glutamate receptors. The pathway is present both at the periphery and in the brain. Main products of this pathway are: the quinolinic acid (QUIN), the kynurenic acid (KYNA), and the 3-hydroxykynurenine (3OH-Kyn) that generates free radicals. QUIN is shown to activate NMDA receptors, to induce seizure and to produce excitotoxic lesions. KYNA at high concentrations (µM range) antagonizes the glycine site of the NMDA receptor complex, at mM concentrations antagonizes AMPA and kainate receptors and has neuroprotective effects (Schwarcz and Pellicciari, 2002, JPET 303:1-10).

Kynurenine 3-hydroxylase (Kyn-3-OHase) is a key enzyme of the kynurenine pathway; its inhibition causes a decrease of QUIN and 3OH-Kyn and an increase of KYNA levels.

Kynurenine 3-hydroxylase inhibitors have been proposed as therapeutic agents for the treatment of neurodegenerative disease such Huntington's chorea, Alzheimer's disease, dementia caused by Acquired Immunodeficiency Syndrome (AIDS), infarctual dementia, cerebral ischemia, cerebral hypoxia, Parkinson's disease, epilepsy, head and spinal cord injury, amyotrophic lateral sclerosis, glaucoma retinopathy, infections of the brain or inflammations of the brain.

RO-618048 compound is an inhibitor of Kyn-3OHase (Rover et al, 1997, J. Med. Chem 40:4378-4385). Other kynurenine 3-hydroxylase inhibitors have been disclosed, *inter alia,* in US 6323240, WO 98/40344, WO 98/09938, EP 819681, WO 99/6375, WO 99/28309, WO 99/28316, WO 99/6375, WO 98/3469, WO 95/3271.

### DESCRIPTION OF THE INVENTION

It has now been found that kynurenine 3-hydroxylase inhibitors are useful as therapeutic agents for the treatment of motor disorders associated to chronic L-DOPA treatment, drug addiction, pain and of cataract.

The activity of kynurenine 3-hydroxylase inhibitors has been shown by suitable animal models and experimental evidence, as reported hereinafter.

### Movement disorders associated to chronic L-DOPA treatment

The most widely used treatment of motor disorders in PD is the replacement therapy with the dopamine precursor, levodopa (L-DOPA). However long term treatment with L-DOPA is associated with the desensitization to the effect of L-DOPA (wearing-off phenomenon) and development of motor response complication (Late Motor Complication LMC) and dyskinesia.

In 6-OHDA lesioned rats, a commonly used model of PD, motor response to L-DOPA decreases after chronic treatment. This effect mimics the "wearing-off" phenomenon observed in parkinsonian patients under chronic treatment with L-DOPA (Boldry *et al.,* Brain Res 1995 Sep 18; 692: 259-64).

In this rat model we evaluated the effect of Ro-618048 (40 mg/kg; *ip*) and of 2-(3,4-Dichloro-benzoyl)-cyclopropanecarboxylic acid (UPF-648, 50 mg/kg; *ip*), on the rotational response to chronic L-DOPA (28 days treatment, 25 mg/kg/day; *ip*) in comparison to MK-801 (0.1 mg/kg; *ip*).

### Methods

### 6-Hydroxydopamine-induced lesions

Unilateral lesions of the nigrostriatal pathway were produced by injecting 6-hydroxydopamine (6-OHDA-HBr) into the left medial forebrain bundle (MFB). Male Wistar rats (250-275 g), under sodium pentobarbital (45 mg/kg ip) anesthesia, were placed on a stereotaxic frame (David Kopf inst.). Holes were bored into the skull to allow the placement of an injection cannula at the following coordinates; 4.0 mm anterior to lambda, 1.3 mm left of the midline, and 8.4 mm below the surface of the skull. 6-OHDA-HBr was dissolved in 0.02% ascorbate saline (8 g/4 l) and injected with a Hamilton syringe at a rate of 0.5 µl/min for 8 min (total volume of 4.0 µl). After this procedure, the animals were returned to their home cages for three weeks before behavioral testing.

### Measurement of rotational behavior

To measure rotational behavior, rats were placed in circular cages and tethered to an automated rotometer. The number of complete turns performed in each 5-min period was recorded by a computer. Three weeks after injection of 6-OHDA the animals were screened for rotational behavior by measuring their response to apomorphine (0.05 mg/kg *sc*). Only animals which responded with 100 or more rotations were used.

### Treatments

Rats were injected intraperitoneally twice a day at 8:00 and 16:00 for 28 consecutive days with either a combination of L-DOPA methylester (50 mg/kg) and the peripheral decarboxylase inhibitor benserazide (12.5 mg/kg) or saline. Rotation was screened at the beginning of treatment (L-DOPA day 1) and the end of treatment (L-DOPA day 28), and the following day when rats also received drugs (Ro-618048, MK801). Eight rats for each group were used. The duration of the response was measured by the time between the first 5 min interval when the rate of turning attained half its eventual average value and the first interval when the rate again declined to half its average values. Statistical analysis

Data were analyzed by analysis of variance followed by Duncan's new multiple range test for post-hoc comparisons.

### Results

The rotational response to L-DOPA became progressively shorter during the course of 4 weeks treatment, declining by 20% (p<0.05) on day 28 of L-DOPA treatment in comparison with rotation measured at day 1.

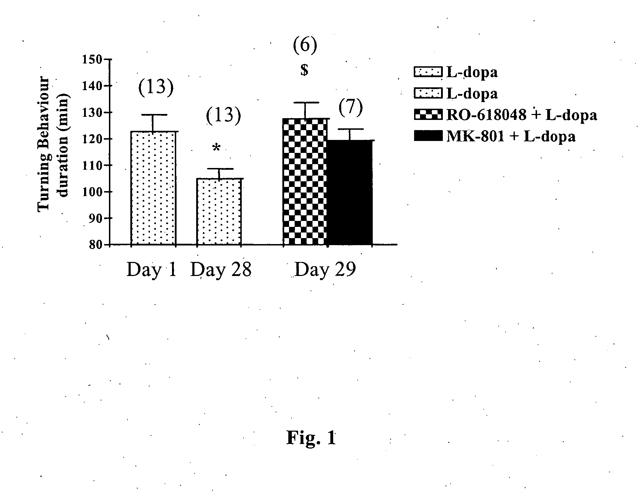

This decreased response to L-DOPA was reversed on the 29th day by acute co-administration of Ro-618048 (40 mg/kg *ip*). This effect was similar to the one obtained with MK801 (0.1 mg/kg *ip*), as shown in figure 1, wherein the bars represent mean values ± SEM; n = number of rats; * and $ indicate P<0.05 significance vs L-dopa treatment on day 1 and 28 respectively. Similar results were obtained with the compound UPF-648 (50mg/kg *ip*).

These results show the ability of the kynurenine-3-hydroxylase inhibitors RO-618048 and UPF-648 to reverse the decreased response to chronic L-DOPA. These data show that kynurenine-3-hydroxylase inhibitors may be used for the treatment of movement disorders associated to chronic L-DOPA treatment in PD patients and in dyskinesias.

### Drug addiction

Drug addiction is a pathological behavior characterized by compulsive drug seeking and intake (Koob et al, 1998, *Neuron* **21,** 467-476). Continued drug use is believed to cause protracted functional changes in the neural circuits involved in motivation that can lead to dependence, drug craving and relapse (Koob et al, 1998, *Neuron* **21,** 467-476). One animal model of these behavioral changes is the long-lasting increase in locomotor activity induced by repeated administration of psychostimulant drugs in rodents known as drug-induced behavioral sensitization (Robinson and Berridge, 1993, *Brain Res Rev* **18,** 247-91 1993).

We evaluated the effect of Ro-618048 (40 mg/kg; *ip*) and of 2-(3,4-Dichloro-benzbyl)-cyclopropanecarboxylic acid (UPF-648, 50 mg/kg; *ip*) in a model of cocaine-induced behavioral sensitization in rat. The results demonstrate that Ro-618048 and UPF-648 prevent the induction of behavioral sensitization to cocaine.

### Methods

### Locomotor activity apparatus

Male Wistar rats (Charles River, Kingston, NY) weighing 200-250 g upon arrival were used. Locomotor activity was measured in sixteen identical metal wire hanging cages each measuring 36 cm (L) x 25 cm (W) x 20 cm (H). Each cage contained two sets of infrared emitter-detector photocells positioned along the long axis 1-cm above the grid floor and 8 cm from the front and back of the cage. Background noise was provided by a white noise generator. Movement within the cages produced photocell interruptions, which were automatically recorded by an IBM-compatible computer.

### Sensitization procedure and treatment

Animals were habituated to the locomotor activity chambers for 2-3 consecutive days before the experiment. Rats received 5 daily IP injections of cocaine (15 mg/kg) or saline and either the Kyn-3OHase inhibitor Ro-618048 (40mg/kg ip) or its vehicle and locomotor activity was recorded for 3 hours. Ten days after the last injection of cocaine or saline (day 15), the animals were challenged with 15 mg/kg of cocaine in absence of Ro-618048 and locomotor activity was again monitored for 3h.

### Statistical analysis

The data (total number of beam breaks in 3 hours) were analyzed using a two way ANOVA with repeated measures on one factor including the four experimental groups (i.e., saline/vehicle, saline/Ro-618048, cocaine/vehicle and cocaine/Ro-618048) and two time points (day 1 and day 5) followed by a simple effects analysis. A second two way ANOVA with repeated measures on one factor was used to compare day 1 and the challenge day followed by a Newman-Keuls post hoc test.

### Results

By the fifth day of treatment with cocaine, animals pretreated IP with vehicle showed an increased locomotor response (20% higher then the first day, p < 0.05), on the contrary, rats pretreated with Ro-618048 (40 mg/kg IP) did not show any significant increase. Ten days after the last injection of cocaine or saline, the animals were challenged with 15 mg/kg of cocaine in absence of the Ro-618048 and locomotor activity was again monitored for 3h. As expected, the rats previously treated with cocaine and that had not received Ro-618048 showed increased locomotor activity response to cocaine (30% higher then first day, p <0.05) but rats that had been pretreated with Ro-618048 during the 5 day-cocaine treatment did not. Similar results were obtained with UPF-648 (50mg/kg *ip*).

These data show that Ro-618048 and UPF-648 prevent the development of locomotor sensitization to cocaine. Kynurenine-3-hydroxylase inhibitors may accordingly be used in the treatment of drug abuse addiction and dependence. ,

### PAIN

Acute and chronic pain begin with and activation of nociceptive pathways by a noxious stimulus. Both types of pain serve a survival function by leading to behavioural and reflexive protection of injured tissue. However chronic pain can become autonomous from the injured tissue and become disabling. Indeed, chronic and neuropathic pain associated with prolonged tissue damage or injury to the peripheral or central nervous system (CNS) are the result of a number of complex changes occurring at various levels in nociceptive pathways. In particular, after inflammation of injured tissue or nerve injury, a tonic input from C nociceptive fibres produces a hyperexcitable state among central nociceptive pathways resulting in hyperalgesia, allodynia, and spontaneous pain (Price, 1996 Pain; 68: 1-3).

It has been shown that tryptophan and some of its metabolites have analgesic properties in animal models (Heyliger et al, 1998 Pharmacol Res 38: 243-50).

We evaluated the analgesic activity of Ro-618048 (40 mg/kg; *ip*) and of 2-(3,4-Dichloro-benzoyl)-cyclopropanecarboxylic acid (UPF-648, 50 mg/kg; *ip*) in animal models of inflammatory and neuropathic pain (formalin test in mice) as well as in acute pain test (tail flick in rats).

### Methods

### Animals

Adult male Wistar rats weighing 175-200 g, and Cr1:CD-1 mice weighing 22-25 g at testing were used (Harlan-Nossan, Italy).

### Mice Formalin Test.

The formalin test in mouse is a procedure used to model neurogenic inflammation and continuous pain (Shibata et al., 1989, Pain 38: 347-352) as well as some features of post-injury pain in man (Dallel et al., 1995, Pain. 61: 11-16).

According to a modified protocol from Rosland et al., (1990, Pain 42: 235-242) mice were injected subcutaneously (s.c.) with 20 µl of 2.7% solution of formalin into the plantar surface of left hindpaw and placed immediately into clear PVC observation chambers (23 x 12 x 13 cm). Pain behavior was quantified by counting the cumulative licking time (sec) of the injected paw. Measurements were taken during the early phase (0-5 min) and late phase (30-40 min) after formalin injection (Tjolsen et al.,1992, Pain 51: 5-17). Ro-618048 40 mg/kg was administered ip 15 min before formalin injection in a volume of 2 ml/kg body weight to groups of 10 mice per dose. Control group was treated with vehicle.

### Tail Flick Test

The tail flick test measures the thermal nociceptive threshold defined as the latency required to elicit a tail response to heat. The test was performed according to the method described by D'Amour and Smith (1941, J. Pharmacol. Exp. Ther. 72:74-79) using an analgesiometer (U. Basile, Italy). Rats were treated ip with the drug (Ro-618048 40 mg/kg) or vehicle and 90 min after, each animal was placed on a platform and the tail was exposed to a focused beam of radiant heat approximately 3 cm from the tip. The beam light intensity was adjusted to produce a reaction latency of 3-5 sec in control rats. The thermal nociceptive threshold was defined as the latency required, to elicit a tail response. A 20 sec cut-off was used to prevent tissue damage.

### Data Analysis and Statistics

Mice Formalin test. Data are presented as mean ± SEM of 8-10 animals per dose group. Data were evaluated by analysis of variance followed by Dunnett's t-test.

The cumulative licking time (sec) of the injected paw was recorded during the early phase (0-5 min) and late phase (30-40 min) after formalin injection.

Tail Flick Test. The latency was defined as the interval of time (sec) between the onset of the thermal stimulus and the flick of the tail. Data were evaluated by analysis of variance followed by Dunnett's t-test.

### Results

Effect in Mice Formalin Test: Ro-618048 at the dose of 40 mg/kg significantly reduced cumulative licking time in the early phase. The cumulative licking time was reduced from 106 ± 4.39 sec (control group) to 75 ± 5.5 sec (p<0.05).

In the late phase, Ro-618048 caused a significant (p<0.05) dose-dependent reduction of the cumulative licking time. The cumulated licking time was reduced from 123±12.8 sec in the control group to 68±14.3 sec. Similar results were obtained with UPF-648 (50mg/kg *ip*).

Effect in tail flick test: two groups of rats received ip vehicle or Ro-618048 (40 mg/kg). In the control group the tail flick response latency to noxious heat stimuli was 3.9±0.4 sec. In the treated group, the latency was statistically different (6.1±0.4 sec) 90 min after administration. Similar results were obtained with UPF-648 (50mg/kg *ip*).

Our results show that Ro-618048 and UPF-648 have an antinociceptive effect in the used models. These data show that the use of Kyn 3OHase inhibitors may be beneficial in the treatment of acute, inflammatory, neuropathic and chronic pain in humans.

### CATARACT

The mammalian lens contains elevated concentrations of 3-hydroxykynurenine (3OH-Kyn), one of the tryptophan (TRP) metabolites formed along the "kynurenine metabolic pathway". Two enzymes are necessary for 3OH-Kyn synthesis from TRP: the first is indoleamine-2,3-dioxygenase (IDO) which is able to metabolise TRP into kynurenine; the second is Kynurenine 3-Hydroxylase (Kyn-3-OHase) which metabolises kynurenine into 3OH-Kyn. Both of them are highly expressed in the iris/ciliary body where a significant amount of 3OH-Kyn is synthesized Chiarugi *et al.* 1999, FEBS Lett. 18;453(1-2):197-200). It has been proposed that neo-formed 3OH-Kyn is then continuously released into the aqueous humor and actively taken up into the lens, where it may be used for the synthesis of UV-filtering compounds. It is now widely accepted that 3OH-Kyn itself, its glycoside derivative and the glycoside derivatives of 4-(2-amino-3-hydroxyphenyl)-4-oxobutanoic acid, one of 3OH-Kyn metabolites, are effective sunlight filters and their presence in the lens of diurnal animals could be useful in protecting the retina from UV radiation present in the sun light (Truscott *et al.* 1994, Dev. Ophthalmol. 26:83-6.; Taylor *et al* 2001, Br. J. Ophthalmol. Sep;85(9):1120-6).

Besides protecting the retina from UV light, accumulation of 3OH-Kyn and several of its metabolites in the lens facilitates the processes leading to lens opacification and senile cataract formation. In fact, 3OH-Kyn reacts with lens proteins and forms tanned products resembling those found in cataract material. Furthermore, the o-aminophenolic moiety of 3OH-Kyn and related compounds undergo complex autoxidative processes responsible for the formation of radical species that are able to react with crystallins, to modify protein tertiary structures thus contributing to the oxidative damage of lens proteins and the formation of senile cataracts (Goldstein *et al.,* 2000 Biochemistry. Jun 20;39(24):7266-75). Inhibitors of Kyn-3OHase reduce 3OH-Kyn synthesis in the iris-ciliary body and in the lens, decrease 3HK effects on lens proteins and may prevent the occurrence of senile cataracts.

We evaluated the effect of a treatment with inhibitors of Kyn-3OHase (Ro-618048) and of 2-(3,4-Dichloro-benzoyl)-cyclopropanecarboxylic acid (UPF-648, 50 mg/kg; *ip*) in an *in vivo* (chronic naphtalene treatment) and an *in vitro* models of cataract (culture lenses exposed to different insults). The results obtained show that the treatment with Kyn-3OHase inhibitors reduce the degree of opacification of the lens in these models.

For the considered therapeutic uses, kynurenine-3-hydroxylase inhibitors will be administered by the oral, parenteral or topical route, suitably formulated in pharmaceutical compositions. The dosage regimen may be adjusted by a skilled practitioner on the basis of the pharmacokinetics and toxicological properties of the selected compound, of the patient's conditions and on the kind of pathology to be treated. It may be presumed, anyhow, that the dosage range will not be substantially different from that already disclosed, e.g. in the above cited prior art patent documents, which are herein incorporated by reference, for the known use in the treatment of neurodegenerative diseases.

## Claims

1. Use of kynurenine-3-hydroxylase inhibitors for the preparation of medicaments for the treatment of L-DOPA induced movement disorders, dyskinesias, drug addiction, pain and cataract.

2. Use according to claim 1 wherein the kynurenine-3-hydroxylase inhibitor is RO-618048 or UPF-648.
